# EUROPEAN PATENT APPLICATION

(11) **EP 1 614 428 A1**
(43) Date of publication of application: **11.01.2006**
(21) Application number: 04712221.3
(22) Date of filing: 18.02.2004
(51) Int. Cl.: A61K 45/00, A61K 31/4178, A61P 27/02, A61P 43/00

(54) **DRUG FOR PREVENTING OR TREATING ANGIOGENIC EYE DISEASES**

(30) Priority: 15.04.2003 JP 2003109918
(71) Applicant: Sankyo Company, Limited, Tokyo 103-8426 (JP)
(72) Inventor: YOKOYAMA, Tomihisa c/o Sankyo Company, Limited, Tokyo 1408710 (JP); INOUE, Tatsuya c/o Sankyo Company, Limited, Tokyo 1408710 (JP)
(74) Representative: Gibson, Christian John Robert
(86) International application number: PCT/JP2004/001818
(87) International publication number: WO 2004/091659

(57) **Abstract**

A medicament for the prevention or treatment of intraocular angiogenic diseases such as proliferative retinopathy, retinal vein occlusion, retinal artery occlusion or age-related macular degeneration, which comprises an angiotensin II receptor antagonist such as 4-(1-hydroxy-1-methylethyl)-2-propyl-1-[2'-(1H-tetrazol-5-yl)biphenyl 4-ylmethyl]imidazole-5-carboxylic acid as its active ingredient.

## Description

### [Technical field]

The present invention relates to a medicament for the prevention or treatment of intraocular angiogenic diseases comprising an angiotensin II receptor antagonist as its active ingredient.

### [Background art]

Angiotensin II receptor antagonists are used as therapeutic agents of circulatory diseases including heart diseases such as cardiac hypertrophy, cardiac failure or myocardial infarction, cerebral apoplexy or nephritis, as well as hypertension, and it is thought that their mechanism of action is the result of inhibiting binding of angiotensin II, which has potent vasoconstrictive action, to angiotensin II receptors.

The actions of angiotensin II receptor antagonists on intraocular angiogenic diseases are known to involve angiotensin II receptor antagonists improving changes in electroretinograms in an animal model of diabetes (see, for example, Diabetologia, 44, 883-888, 2001), and suppressing neovascularization of the retina in an animal model of proliferative retinopathy (see, for example, Investigative Ophthalmology & Visual Science, 42, 429-432, 2001). In addition, compounds having angiotensin II antagonistic action are known to be effective in the prevention or treatment of simple retinopathy and pre-proliferative retinopathy, which are early and intermediate disease states of diabetic retinopathy (see, for example, Japanese Patent Application (Kokai) No. 2001-10975). However, it has not conventionally been known that compounds having angiotensin II antagonistic action are effective in the prevention or treatment of intraocular angiogenic diseases such as proliferative retinopathy, which occurs in the late stage of diabetic retinopathy or retinal vein occlusion. Furthermore, vascular endothelial growth factor (VEGF) is considered to play an important role as a regulatory factor of neovascularization in intraocular angiogenic diseases (Molecular Medicine, 35, 1252-1260, 1998, Journal of Cellular Physiology, 184, 301-310, 2000).

### [Disclosure of the invention]

An object of the present invention is to provide a useful medicament for the prevention or treatment of retinal ischemia. As a result of extensive research to achieve the object described above, the inventors of the present invention have found that an angiotensin II receptor antagonist is highly effective in the prevention or treatment of intraocular angiogenic diseases such as proliferative retinopathy or retinal vein occlusion. The present invention was completed based on these findings described above.

Specifically, the present invention provides a medicament for the prevention or treatment of intraocular angiogenic diseases comprising an angiotensin II receptor antagonist as its active ingredient. According to a preferred embodiment of this invention, the medicament described above is provided in which the intraocular angiogenic disease is proliferative retinopathy, retinal vein occlusion, retinal artery occlusion or age-related macular degeneration.

According to a more preferred embodiment of the present invention, the medicament described above is provided in which the angiotensin II receptor antagonist is selected from the group consisting of 4-(1-hydroxy-1-methylethyl)-2-propyl-1-[2'-(1H-tetrazol-5-yl)biphenyl-4-ylmethyl]imidazole-5-carboxylic acid, pharmacologically acceptable esters thereof and pharmacologically acceptable salts thereof, and according to a further more preferred embodiment, the medicament described above is provided in which the angiotensin II receptor antagonist is (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate.

In another aspect, the use of an angiotensin II receptor antagonist, preferably the use of an angiotensin II receptor antagonist selected from the group consisting of 4-(1-hydroxy-1-methylethyl)-2-propyl-1-[2'-(1H-tetrazol-5-yl)biphenyl-4-ylmethyl]imidazole-5-carboxylic acid, pharmacologically acceptable esters thereof and pharmacologically acceptable salts thereof, and more preferably the use of (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate, to manufacture the medicament described above are provided by the present invention.

In another aspect, methods for the prevention or treatment of intraocular angiogenic diseases, and preferably proliferative retinopathy, retinal vein occlusion, retinal artery occlusion or age-related macular degeneration, comprising any mode of administration of preventive or therapeutically effective doses of an angiotensin II receptor antagonist, preferably an angiotensin II receptor antagonist selected from the group consisting of 4-(1-hydroxy-1-methylethyl)-2-propyl-1-[2'-(1H-tetrazol-5-yl)biphenyl-4-ylmethyl]imidazole-5-carboxylic acid, pharmacologically acceptable esters thereof and pharmacologically acceptable salts thereof, and more preferably (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate, to mammals, including humans, are provided by the present invention.

### [Best mode for carrying out the invention]

In the present invention, an angiotensin 11 receptor antagonist refers to a substance capable of competitively or non-competitively inhibiting the binding of angiotensin II to an angiotensin II receptor. The angiotensin II receptor antagonist may be a low molecular weight organic compound, a peptide compound, a saccharide compound or a high molecular weight compound such as a protein, glycoprotein or polysaccharide compound. Whether or not a certain substance has angiotensin II receptor antagonistic action can easily be confirmed by a person with ordinary skill in the art in accordance with, for example, the method described in the specification of US Patent No. 5,616,599. The term "angiotensin II receptor antagonist" used in the present specification should not be interpreted in a limiting manner, but rather should be interpreted in the broadest sense for all meanings for which it is used.

As examples of angiotensin II receptor antagonists which can be preferably used in the medicament of the present invention, 4-(1-hydroxy-1-methylethyl)-2-propyl-1-[2'-(1H-tetrazol-5-yl)biphenyl-4-ylmethyl)imidazole-5-carboxylic acid, pharmacologically acceptable esters thereof and pharmacologically acceptable salts thereof can be listed. 4-(1-Hydroxy-1-methylethyl)-2-propyl-1-[2'-(1H-tetrazol-5-yl)biphenyl-4-ylmethyl]imidazole-5-carboxylic acid is known, and can easily be obtained by the method described in, for example, Japanese Patent Application (Kokai) No. Hei 5-78328 (US Patent No. 5,616,599).

The pharmacologically acceptable salts of 4-(1-hydroxy-1-methylethyl)-2-propyl-1-[2'-(1H-tetrazol-5-yl)biphenyl-4-ylmethyl]imidazole-5-carboxylic acid are not restricted, and these salts can be selected by a person with ordinary skill in the art, for example, an alkali metal salt such as sodium salt, potassium salt or lithium salt, an alkaline earth metal salt such as calcium salt or magnesium salt, a metal salt such as aluminium salt, iron salt, zinc salt, copper salt, nickel salt or cobalt salt; or an amine salt such as ammonium salt, t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt, tris(hydroxymethyl)aminomethane salt or the like. Preferably alkali metal salts can be used, and particularly preferably the sodium salt can be used.

The pharmacologically acceptable esters of 4-(1-hydroxy-1-methylethyl)-2-propyl-1-[2'-(1H-tetrazol-5-yl)biphenyl-4-ylmethyl]imidazole-5-carboxylic acid refer to the compound of which an intramolecular carboxyl moiety is esterified. The pharmacologically acceptable esters are not restricted, and these esters can be selected by a person with ordinary skill in the art. It is preferable that the said esters can be cleaved by a biological process such as hydrolysis in vivo. As the group constituting the said ester moiety (the group shown as R when the ester moiety is expressed as -COOR), for example, a C₁-C₄ alkoxy C₁-C₄ alkyl group such as methoxymethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, propoxymethyl, isopropoxymethyl, butoxymethyl or t-butoxymethyl; a C₁-C₄ alkoxylated C₁-C₄ alkoxy C₁-C₄ alkyl group such as 2-methoxyethoxymethyl; a C₆-C₁₀ aryloxy C₁-C₄ alkyl group such as phenoxymethyl; a halogenated C₁-C₄ alkoxy C₁-C₄ alkyl group such as 2,2,2-trichloroethoxymethyl or bis(2-chloroethoxy)methyl; a C₁-C₄ alkoxycarbonyl C₁-C₄ alkyl group such as methoxycarbonylmethyl; a cyano C₁-C₄ alkyl group such as cyanomethyl or 2-cyanoethyl; a C₁-C₄ alkylthiomethyl group such as methylthiomethyl or ethylthiomethyl; a C₆-C₁₀ arylthiomethyl group such as phenylthiomethyl or naphthylthiomethyl; a C₁-C₄ alkylsulfonyl C₁-C₄ lower alkyl group which may optionally be substituted with a halogen atom(s) such as 2-methanesulfonylethyl or 2-trifluoromethanesulfonylethyl; a C₆-C₁₀ arylsulfonyl C₁-C₄ alkyl group such as 2-benzenesulfonylethyl or 2-toluenesulfonylethyl; a C₁-C₇ aliphatic acyloxy C₁-C₄ alkyl group such as formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl or 1-pivaloyloxyhexyl; a C₅-C₆ cycloalkylcarbonyloxy C₁-C₄ alkyl group such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl or 1-cyclohexylcarbonyloxybutyl; a C₆-C₁₀ arylcarbonyloxy C₁-C₄ alkyl group such as benzoyloxymethyl; a C₁-C₆ alkoxycarbonyloxy C₁-C₄ alkyl group such as methoxycarbonyloxymethyl, 1-(methoxycarbonyloxy)ethyl, 1-(methoxycarbonyloxy)propyl, 1-(methoxycarbonyloxy)butyl, 1-(methoxycarbonyloxy)pentyl, 1-(methoxycarbonyloxy)hexyl, ethoxycarbonyloxymethyl, 1-(ethoxycarbonyloxy)ethyl, 1-(ethoxycarbonyloxy)propyl, 1-(ethoxycarbonyloxy)butyl, 1-(ethoxycarbonyloxy)pentyl, 1-(ethoxycarbonyloxy)hexyl, propoxycarbonyloxymethyl, 1-(propoxycarbonyloxy)ethyl, 1-(propoxycarbonyloxy)propyl, 1-(propoxycarbonyloxy)butyl, isopropoxycarbonyloxymethyl, 1-(isopropoxycarbonyloxy)ethyl, 1-(isopropoxycarbonyloxy)butyl, butoxycarbonyloxymethyl, 1-(butoxycarbonyloxy)ethyl, 1-(butoxycarbonyloxy)propyl, 1-(butoxycarbonyloxy)butyl, isobutoxycarbonyloxymethyl, 1-(isobutoxycarbonyloxy)ethyl, 1-(isobutoxycarbonyloxy)propyl, 1-(isobutoxycarbonyloxy)butyl, t-butoxycarbonyloxymethyl, 1-(t-butoxycarbonyloxy)ethyl, pentyloxycarbonyloxymethyl, 1-(pentyloxycarbonyloxy)ethyl, 1-(pentyloxycarbonyloxy)propyl, hexyloxycarbonyloxymethyl, 1-(hexyloxycarbonyloxy)ethyl or 1-(hexyloxycarbonyloxy)propyl; a C₅-C₆ cycloalkyloxycarbonyloxy C₁-C₄ alkyl group such as cyclopentyloxycarbonyloxymethyl, 1-(cyclopentyloxycarbonyloxy)ethyl, 1-(cyclopentyloxycarbonyloxy)propyl, 1-(cyclopentyloxycarbonyloxy)butyl, cyclohexyloxycarbonyloxymethyl, 1-(cyclohexyloxycarbonyloxy)ethyl, 1-(cyclohexyloxycarbonyloxy)propyl or 1-(cyclohexyloxycarbonyloxy)butyl; a [5-(C₁-C₄ alkyl)-2-oxo-1,3-dioxolen-4-yl]methyl group such as (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl or (5-butyl-2-oxo-1,3-dioxolen-4-yl)methyl; a [5-(phenyl, which may optionally be substituted with a C₁-C₄ alkyl, C₁-C₄ alkoxy or halogen atom(s))-2-oxo-1,3-dioxolen-4-yl]methyl group such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl or [5-(4-chlorophenyl)-2-oxo-1,3-dioxolen-4-yl]methyl; or a phthalidyl group, which may optionally be substituted with a C₁-C₄ alkyl or C₁-C₄ alkoxy group(s), such as phthalidyl, dimethylphthalidyl or dimethoxyphthalidyl can be listed. Preferably a pivaloyloxymethyl group, phthalidyl group or (5-methyl-2-oxo-1,3-dioxolen-4-yl)methyl group can be used, and more preferably a (5-methyl-2-oxo-1,3-dioxolen-4-yl)methyl group can be used.

In the case that esters of 4-(1-hydroxy-1-methylethyl)-2-propyl-1-[2'-(1H-tetrazol-5-yl)biphenyl-4-ylmethyl]imidazole-5-carboxylic acid form pharmacologically acceptable salts, the pharmacologically acceptable salts can be selected by a person with ordinary skill in the art, and are not particularly restricted. Such salts can be, for example, a hydrohalogenic acid salt such as a hydrofluoride, hydrochloride, hydrobromide or a hydroiodide; a nitrate; a perchlorate; a sulfate; a phosphate; a C₁-C₄ alkanesulfonic acid salt, which may optionally be substituted with a halogen atom(s) such as a methanesulfonate, trifluoromethanesulfonate or ethanesulfonate; a C₆-C₁₀ arylsulfonic acid salt, which may optionally be substituted with a C₁-C₄ alkyl group(s) such as a benzenesulfonate or p-toluenesulfonate; a C₁-C₆ aliphatic acid salt such as an acetate, malate, fumarate, succinate, citrate, tartrate, oxalate or maleate; or an amino acid salt such as a glycine salt, a lysine salt, an arginine salt, an ornithine salt, a glutamic acid salt and an aspartic acid salt. Preferably a hydrochloride, nitrate, sulfate or phosphate can be used, and particularly preferably a hydrochloride can be used.

Preferably 4-(1-hydroxy-1-methylethyl)-2-propyl-1-[2'-(1H-tetrazol-5-yl)biphenyl-4-ylmethyl]imidazole-5-carboxylic acid or pharmacologically acceptable esters thereof can be used as the angiotensin II receptor antagonist, more preferably pharmacologically acceptable esters of 4-(1-hydroxy-1-methylethyl)-2-propyl-1-[2'-(1H-tetrazol-5-yl)biphenyl-4-ylmethyl]imidazole-5-carboxylic acid can be used, and further more preferably a pivaloyloxymethyl ester, phthalidyl ester or (5-methyl-2-oxo-1,3-dioxolen-4-yl)methyl ester of 4-(1-hydroxy-1-methylethyl)-2-propyl-1-[2'-(1H-tetrazol-5-yl)biphenyl-4-ylmethyl]imidazole-5-carboxylic acid can be used. Most preferably, (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate can be used.

As the compound selected from the group consisting of 4-(1-hydroxy-1-methylethyl)-2-propyl-1-[2'-(1H-tetrazol-5-yl)biphenyl-4-ylmethyl]imidazole-5-carboxylic acid, pharmacologically acceptable esters thereof and pharmacologically acceptable salts thereof, their hydrates or solvates can also be used.

In the case that the pharmacologically acceptable esters of 4-(1-hydroxy-1-methylethyl)-2-propyl-1-[2'-(1H-tetrazol-5-yl)biphenyl-4-ylmethyl]imidazole-5-carboxylic acid are used, some esterified compounds may have one or two or more asymmetric carbons, and optical isomers purified based on the said asymmetric carbons or stereoisomers such as diastereoisomers or any mixtures of these stereoisomers or racemate can also be used.

In addition, as angiotensin II receptor antagonists that can be used in the present invention, for example, imidazole derivatives (Japanese Patent Application (Kokai) No. Sho 56-71073, Japanese Patent Application (Kokai) No. Sho 56-71074, Japanese Patent Application (Kokai) No. Sho 57-98270, Japanese Patent Application (Kokai) No. Sho 58-157768, USP 4,355,040, USP 4,340,598, EP-253310, EP-291969, EP-324377, EP-403158, WO-9100277, Japanese Patent Application (Kokai) No. Sho 63-23868, Japanese Patent Application (Kokai) No. Hei 1-117876, etc.), pyrrole, pyrazole and triazole derivatives (USP 5,183,899, EP-323841, EP-409332 and Japanese Patent Application (Kokai) No. Hei 1-287071, etc.), benzimidazole derivatives (USP No. 4,880,804, EP-0392317, EP-0399732, EP-0400835, EP-425921, EP-459136, Japanese Patent Application (Kokai) No. 2001-10975, Japanese Patent Application (Kokai) No. Hei 3-63264 etc.), azaindene derivatives (EP-399731, etc.), pyrimidone derivatives (EP-407342, etc.), quinazoline derivatives (EP-411766, etc.), xanthine derivatives (EP-430300, etc.), condensed imidazole derivatives (EP-434038, etc.), pyrimidinedione derivatives (EP-442473, etc.), thienopyridone derivatives (EP-443568, etc.), heterocyclic compounds (EP-445811, EP-483683, EP-518033, EP-520423, EP-588299, EP-603712, etc.) and the like can be listed, and preferably Losartan, Eprosartan, Candesartan cilexetil, Valsartan, Telmisartan, Irbesartan, Tasosartan and their metabolically active compounds can be listed. In addition, pharmacologically acceptable salts of these compounds, hydrates or solvates of their free forms or their salt forms, or derivatives such as prodrugs can also be used.

The medicament of the present invention can be used for the prevention or treatment of intraocular angiogenic diseases. In the present specification, the phrase "prevention or treatment" includes the amelioration or cure of diseases, as well as suppression of the progress or inhibition of the onset of diseases, and the prevention of its recurrence. The phrase "prevention or treatment" should not be interpreted in a limiting manner, but rather should be interpreted in the broadest sense for all meanings for which it is used.

Intraocular angiogenic diseases are the diseases that exhibit pathology primarily consisting of neovascularization within the eye, which include,for example, proliferative retinopathy, retinal vein occlusion, retinal artery occlusion or age-related macular degeneration. Proliferative retinopathy is a form of diabetic retinopathy, and is defined as being a disease state that follows an early disease stage in the form of simple retinopathy, and an intermediate disease stage in the form of pre-proliferative retinopathy (Diabetes, 42, 409-410 (1999)). It can be distinctly classified by experienced physicians. Furthermore, the term "proliferative retinopathy" includes the entire series of disease stages that lead to retinal detachment due to neovascularization within the retina in general.

Namely, the medicament of the present invention comprising an angiotensin II receptor antagonist as its active ingredient can be used for the prevention or treatment of intraocular angiogenic diseases such as simple retinopathy, pre-proliferative retinopathy, vascular disorder retinopathy, arteriosclerotic retinopathy, hypertensive retinopathy, retinopathy of prematurity, renal retinopathy, macular edema and the like.

Since the angiotensin II receptor antagonists described above in general are medicaments to be administered orally, the medicament of the present invention is favorable to being administered orally. However, the administration forms of the medicament of the present invention are not restricted to oral administration, but rather can also be parenteral administration such as intravenous administration, intrarectal administration, transcutaneous administration, transmucosal administration and the like. As suitable dosage forms for oral administration, powders, granules, tablets, capsules and the like can be listed, but are not restricted to these forms. One or two or more types of preparation additives can be used when preparing the dosage forms.

As preparation additives, for example, excipients (for example, organic excipients including sugar derivatives such as lactose, sucrose, glucose, mannitol or sorbitol; starch derivatives such as corn starch, potato starch,-αstarch or dextrin; cellulose derivatives such as crystalline cellulose; gum arabic; dextran; or pullulan; or inorganic excipients including silicate derivatives such as light anhydrous silicic acid, synthetic aluminium silicate, calcium silicate or magnesium aluminometasilicate; phosphates such as calcium hydrogenphosphate; carbonates such as calcium carbonate; or sulfates such as calcium sulfate can be listed), lubricants (for example, stearic acid or metal salts of stearic acid such as calcium stearate or magnesium stearate; talc; waxes such as beeswax or spermaceti wax; boric acid; adipic acid; sulfates such as sodium sulfate; glycol; fumaric acid; sodium benzoate; DL-leucine; laurylsulfates such as sodium lauryl sulfate or magnesium lauryl sulfate; silicates such as silicic anhydride or silicic hydrate; or the starch derivatives described above can be listed), binders (for example, hydroxypropylcellulose, hydroxypropylmethylcellulose, polyvinylpyrrolidone, macrogol or similar excipients to those described above can be listed), disintegrants (for example, cellulose derivatives such as low-substituted hydroxypropylcellulose, carboxymethylcellulose, calcium carboxymethylcellulose or internally crosslinked sodium carboxymethylcellulose; or chemically modified starch/cellulose derivatives such as carboxymethylstarch, sodium carboxymethylstarch or bridged polyvinylpyrrolidone; or the starch derivatives described above can be listed), emulsifiers (for example, colloidal clays such as bentonite or veegum; metal hydroxides such as magnesium hydroxide or aluminium hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose esters of fatty acids can be listed), stabilizers (for example, paraoxybenzoate esters such as methyl paraben or propyl paraben; alcohols such as chlorobutanol, benzyl alcohol or phenylethyl alcohol; benzalkonium chloride; phenols such as phenol or cresol; thimerosal; dehydroacetic acid; or sorbic acid can be listed), flavour and odour correctives (for example, commonly used sweeteners, sour flavourings or fragrances can be listed) or diluents and the like can be listed, but are not restricted to these additives.

Although the dosage of the medicament of the present invention can be selected in a suitable manner depending on various factors such as administration routes, types of active ingredients, patients' age, body weight or symptom, purposes of the prevention or treatment and the like, an adult daily dosage that can be administered as the weight of the angiotensin II receptor antagonist is in general within the range of about 0.001 to 1,000 mg, and preferably about 0.1 to 500 mg.

### [Examples]

The present invention will be hereinafter described in more detail by the Examples and Preparation examples, but the scope of the present invention should not be limited to the following examples.

### Example 1

Seven-day-old C57BL/6 mice were housed for five days with their mother in roughly 75% oxygen. Subsequently, the animals were housed for another five days after returning to ordinary air. While housing in air, the animals were administered a test compound once a day. Following completion of administration, the animals were sacrificed by dislocation of the cervical vertebra followed by excision of the eyeball. The eyeballs were fixed in neutral formalin. After preparing flat specimens of the retina, vascular endothelium was stained by ADPase staining (Lutty, et al., Arch. Ophthalmol. 1992; 100: 267). After staining, retinopathy was scored in a blind study, and retinopathy was assessed for each of 12 retinal sections obtained by dividing the entire circumference of the retina into 12 equal sections centering about the papilla of the optic nerve. Assessments were made by scoring either the presence of retinopathy (score: 1) or the absence of retinopathy (score: 0), and determining the retinopathy score for each specimen based on the total score of the 12 sections. The score of each test compound was calculated as a percentage based on assigning a value of 100% for the score following administration of solvent. The test compounds consisted of (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate (hereinafter, indicated as "Compound A") for the medicament of the present invention, Candesartan cilexetil and Losartan.

**Table 1**

| Test Compound | Dosage (mg/kg) | Retinopathy (%) | p Value |
|---|---|---|---|
| Control (solvent) | | 100 | |
| Compound A | 1 | 57 ± 13 | p<0.01 |
| | 3 | 40 ± 9 | p<0.001 |
| | 10 | 31 ± 7 | p<0.001 |
| | 30 | 32 ± 11 | P<0.001 |
| Candesartan cilexetil | 1 | 84 ± 14 | n.s. |
| | 3 | 70 ± 12 | n.s. |
| | 10 | 56 ± 11 | p<0.05 |
| | 30 | 58 ± 11 | p<0.05 |
| Losartan | 3 | 90 ± 11 | n.s. |
| | 10 | 54 ± 10 | p<0.01 |
| | 30 | 42 ± 9 | p<0.001 |
| | 100 | 40 ± 11 | p<0.001 |

| | | | |
|---|---|---|---|
| n = 9 to 16 | | | |
| n.s. indicates "not significant", and refers to the absence of statistical significance. | | | |

### Example 2

Male New Zealand White rabbits were used in this example. The animals were anesthetized by administering suitable amounts of animal Ketaral (registered trade mark) 50 and Celactal (registered trade mark). Moreover, the eyes were topically anesthetized by dropping 0.4 % Benoxil (registered trade mark) ophthalmic solution. The procedure was basically performed in accordance with the method of Ziche, et al. (J. Clin. Invest. 1997 99: 2625-2634), and consisted of creating a pocket in the centre of the cornea with a razor, and inserting a hydron pellet containing VEGF produced in advance into the pocket. A solution of the test compound was administered once a day for one week. Corneal neovascularization was photographed on the day after final administration of the test compound. Neovascularization was evaluated using the density and length of newly formed vessels as an index. Namely, neovascularization density was scored from 0 to 5 by referring to standard photographs corresponding to each score. The length of neovascularization was calculated by measuring the length (mm) of vessels uniformly extending from the corneal limbus towards the pellet based on a scale bar on the photograph. The value obtained by multiplying the neovascularization density score by the neovascularization length (mm) was used to indicate angiogenic activity (AA).

**Table 2**

| Test Compound | Dosage (mg/kg) | Angiogenic Activity (AA) | p Value |
|---|---|---|---|
| Control (solvent) | | 0.72 ± 0.15 | |
| Compound A | 10 | 0.14 ± 0.11 | p<0.05 |

| | | | |
|---|---|---|---|
| n = 3 | | | |

| Preparation Example 1 - Capsule | |
|---|---|
| Compound A | 20.0 mg |
| Lactose | 158.7 mg |
| Corn starch | 70.0 mg |
| Magnesium stearate | 1.3 mg |
| Total | 250 mg |

Powders of the above formula were mixed and passed through a 60 mesh sieve followed by filling the powder into a 250 mg No. 3 capsule to prepare the capsule.

| Preparation Example 2 - Tablet | |
|---|---|
| Compound A | 20.0 mg |
| Lactose | 154.0 mg |
| Corn starch | 25.0 mg |
| Magnesium stearate | 1.0 mg |
| Total | 200 mg |

Powders of the above formula were mixed and tableted with a tableting machine to prepare the tablet containing 200 mg per tablet. The tablet can be sugar-coated, if necessary.

### [Industrial applicability]

The medicament of the present invention comprising an angiotensin II receptor antagonist as its active ingredient is useful for the prevention or treatment of intraocular angiogenic diseases such as proliferative retinopathy, retinal vein occlusion, retinal artery occlusion or age-related macular degeneration.

## Claims

1. A medicament for the prevention or treatment of intraocular angiogenic diseases comprising an angiotensin II receptor antagonist as its active ingredient.

2. The medicament according to claim 1, wherein the intraocular angiogenic disease is proliferative retinopathy, retinal vein occlusion, retinal artery occlusion or age-related macular degeneration.

3. The medicament according to claim 1 or 2, wherein the angiotensin II receptor antagonist is selected from the group consisting of 4-(1-hydroxy-1-methylethyl)-2-propyl-1-[2'-(1H-tetrazol-5-yl)biphenyl-4-ylmethyl]imidazole-5-carboxylic acid, pharmacologically acceptable esters thereof and pharmacologically acceptable salts thereof.

4. The medicament according to claim 1 or 2, wherein the angiotensin II receptor antagonist is (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate.
